(19) Europäisches Patentamt
European Patent Office
Office européen des brevets

(11) Veröffentlichungsnummer: **0 056 970**
**B1**

(12) # EUROPÄISCHE PATENTSCHRIFT

(45) Veröffentlichungstag der Patentschrift: **27.03.85**

(51) Int. Cl.⁴: **C 07 D 307/52**, C 07 D 333/20, C 07 C 143/80, A 61 K 31/63

(21) Anmeldenummer: **82100335.7**

(22) Anmeldetag: **19.01.82**

(54) **Basisch substituierte Anthranilsäuren, Verfahren zu ihrer Herstellung und ihre Verwendung.**

(30) Priorität: **22.01.81 DE 3101960**

(43) Veröffentlichungstag der Anmeldung: **04.08.82 Patentblatt 82/31**

(45) Bekanntmachung des Hinweises auf die Patenterteilung: **27.03.85 Patentblatt 85/13**

(84) Benannte Vertragsstaaten: **AT BE CH DE FR GB IT LI LU NL SE**

(56) Entgegenhaltungen:
**DE - A - 1 806 581**
**DE - A - 2 406 972**
**DE - A - 2 453 548**

**MEDICINAL CHEMISTRY, 15, 79-83 (1971)**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Sturm, Karl, Dr., Berndesallee 64, D-6501 Heidesheim (DE)**
Erfinder: **Muschaweck, Roman, Dr., Helmchenweg 39, D-6230 Frankfurt am Main 80 (DE)**
Erfinder: **Hropot, Max, Dr., Friedrich-Stolz-Strasse 13, D-6093 Flörsheim am Main (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung sind Verbindungen der Formel I, die der Gruppe der 5-Sulfamoylanthranilsäuren zuzuordnen sind, sowie deren physiologisch verträgliche Salze.

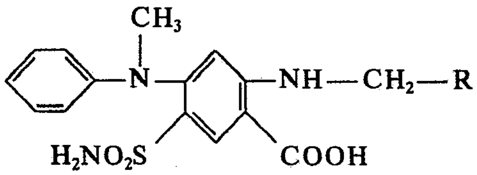

(I)

In der Formel I bedeutet R Furyl, Thienyl oder Phenyl, vorzugsweise 2-Furyl oder 2-Thienyl.

Als Kationen der beanspruchten Salze von I kommen für die therapeutische Verwendung in erster Linie Natrium-, Kalium-, Ammonium- oder substituierte Ammoniumionen in Frage. Besondere Bedeutung haben auch die Salze aus I und einem basischen Arzneimittel wie Antihypertensiva, $\beta$-Blockern oder kaliumretinierenden Substanzen.

Gegenstand der Erfindung ist ferner ein Verfahren zur Herstellung von Verbindungen der Formel I, das dadurch gekennzeichnet ist, daß man ein Nitril der Formel II

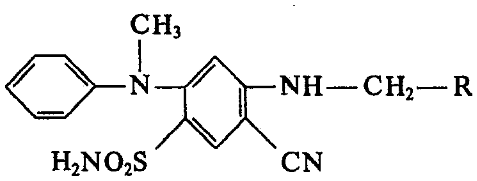

(II)

zur entsprechenden Carbonsäure verseift. Die Verseifung erfolgt vorzugsweise im alkalischen Medium, gegebenenfalls kann sie auch über die Zwischenstufe des aus dem Nitril in üblicher Weise hergestellten Amidrazons, Amidins, Iminoäthers, Amids oder Thioamids geleitet werden.

Das technisch bevorzugte Verfahren ist die direkte Verseifung der Verbindungen der Formel II mit überschüssiger wäßriger Natron- oder Kalilauge unter Rückfluß. Nach beendeter Verseifung werden die Endprodukte vorteilhaft bei pH 3—4 als freie Carbonsäure kristallin gefällt und nach Reinigung durch Umkristallisation nachfolgend gegebenenfalls mit der berechneten Menge Alkalihydroxid, Alkalicarbonat oder Amin in die betreffenden Salze übergeführt.

Besondere pharmakologische Bedeutung haben die Salze der erfindungsgemäßen Verbindungen mit basischen kaliumretinierenden Verbindungen wie beispielsweise Amilorid oder Triamteren oder mit basischen Antihypertensiva wie beispielsweise Clonidin, Dihydralazin oder $\beta$-Blockern. In diesen Salzen kommen die pharmakologischen Eigenschaften beider Komponenten zur Wirkung.

Die Herstellung der als Ausgangsmaterial verwendeten Nitrile der Formel II ist in der älteren Patentanmeldung P 30 41 812.3 (HOE 80/F 254) beschrieben.

Aus der deutschen Offenlegungsschrift 2 453 548 sind 3-Cycloalkylmethylamine-4-anilino-5-sulfamyl-benzoesäuren bekannt, also Verbindungen, die nicht, wie die erfindungsgemäßen, in 2-Stellung den $-NH-CH_2-R$-Rest tragen, und deren 4-Anilinogruppe nicht N-methyliert ist.

2-Benzylamino-4-anilino-5-sulfamoyl-benzoesäure, also eine Verbindung, die sich von denen nach der Erfindung ausschließlich durch die fehlende N-Methylgruppe in der 4-Anilinogruppe unterscheidet, weist eine wesentlich geringere $Na^{(+)}$- und $Cl^{(-)}$-Ausscheidung bei annähernd gleicher $K^{(+)}$-Ausscheidung an Ratten auf. Hinweise auf die erfinderische N-Methylierung finden sich nicht.

Die erfindungsgemäßen Verbindungen sind Salidiuretika vom Furosemid-Typ. Gegenüber Furosemid zeichnen sie sich durch höhere potency, bessere Resorbierbarkeit und eine usicosurische Wirkungskomponente aus.

Sie dienen zur Behandlung cardialer, renaler oder hepatisch bedingter Ödeme, Ascites, Schwangerschaftsödemen, Ödemen nach Verbrennungen und Ödemen nach venöser Insuffizienz oder nach Thrombosen. Ferner auch zur Behandlung von Hypertonien leichten bis mittleren Grades.

Die Anwendung bei Säugetieren und beim Menschen erfolgt vorzugsweise oral oder intravenös, wobei die Dosierungseinheit an reinem Wirkstoff, bezogen auf einen normalgewichtigen erwachsenen Patienten, zwischen 1 und 50 mg liegt.

Für eine orale Anwendungsform werden die aktiven Verbindungen entweder in reiner Form verwendet, oder aber mit den dafür üblichen Zusatzstoffen, wie Trägermitteln, Stabilisatoren oder inerten Verdünnungsmitteln vermischt und durch übliche Methoden in geeignete Darreichungsformen wie beispielsweise Tabletten, Dragees oder Steckkapseln gebracht. Als inerte Träger können z. B. Magnesiumcarbonat, Milchzucker oder Maisstärke verwendet werden. Dabei kann die Zubereitung als Trokken- oder als Feuchtgranulat erfolgen.

Zur intravenösen Applikation werden die aktiven Verbindungen, vorzugsweise in Form ihrer physiologisch verträglichen Alkali- oder Ammoniumsalze, mit den dafür üblichen Substanzen in Lösung

gebracht. Als Lösungsmittel kommt vorzugsweise Wasser gegebenenfalls unter Zusatz von bekannten Puffersubstanzen, Lösungsvermittlern und Stabilisatoren in Frage.

## Beispiele

### Beispiel 1

N-(2-Furymethyl)-4-(N-methylanilino)-5-sulfamoylanthranilsäure

38,2 g 2-Furfurylamino-4-(N-methylanilino)-5-sulfamoylbenzonitril (0,1 Mol) werden mit 0,3 l 2n NaOH 3 Stunden unter Rückfluß erhitzt. Nachfolgend stellt man die Reaktionslösung mit 2n HCl auf pH 8 ein, entfärbt die Lösung mit Aktivkohle und stellt das Filtrat dann mit 2n HCl auf pH 3,0 ein. Nach einstündigem Stehen bei Raumtemperatur wird die kristalline Fällung abgesaugt, gut mit Wasser gewaschen und luftgetrocknet.

Ausbeute: 28,0 g des Trihydrats (62% d. Th.), Schmp. 145° C (u. Gasentw.)

### Beispiel 2

N-(2-Thienylmethyl)-4-(N-methylanilino)-5-sulfamoylanthranilsäure

39,9 g 2-(2-Thienylmethylamino)-4-(N-methylanilino)-5-sulfamoylbenzonitril (0,1 Mol) werden mit 0,3 l 2n NaOH 3 Stunden unter Rückfluß erhitzt und das Endprodukt analog Beispiel 1 als Trihydrat isoliert.

Ausbeute: 31,5 g (67% d. Th.), Schmp. unscharf (ab 80° C Sint.) DC-einheitlich (Silicagel, Methylenchlorid/Methanol 10 : 1, Rf 0,73)

### Beispiel 3

N-Benzyl-4-(N-methylanilino)-5-sulfamoylanthranilsäure

39,3 g 2-Benzylamino-4-(N-methylanilino)-5-sulfamoylbenzonitril (0,1 Mol) werden analog Beispiel 1 mit 0,3 l NaOH verseift und das bei pH 3,0 gefällte, nutschfeuchte Endprodukt aus Isopropanol umkristallisiert. Nach Trocknen auf dem Dampfbad enthält die Verbindung noch 1 Moläquivalent Kristall-Isopropanol.

Ausbeute: 28,5 g (59% d. Th.), Schmp. 128° C (u. Gasentw.)

**Patentansprüche für die Vertragsstaaten: BE, CH, DE, FR, GB, IT, LI, LU, NL, SE**

1. Basisch substituierte Anthranilsäuren der Formel I

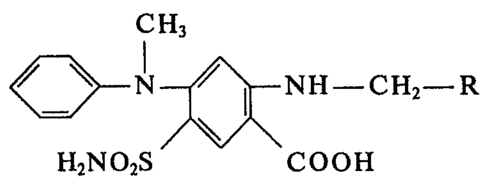

(I)

in der R Furyl, Thienyl oder Phenyl bedeutet und deren physiologisch verträgliche Salze.

2. Anthranilsäuren gemäß Anspruch 1, in denen R 2-Furyl oder 2-Thienyl ist.

3. Salze einer Anthranilsäure gemäß Anspruch 1 mit einer basischen, kaliumretinierenden Verbindung oder einem basischen Antihypertensivum.

4. Verfahren zur Herstellung einer basisch substituierten Anthranilsäure gemäß Anspruch 1, dadurch gekennzeichnet, daß man ein Nitril der Formel II

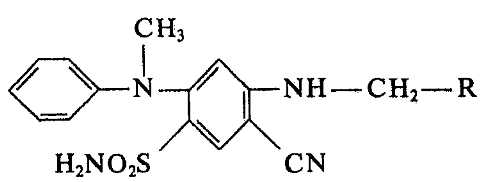

(II)

verseift.

5. Verfahren gemäß Anspruch 4, dadurch gekennzeichnet, daß die Verseifung in einem alkalischen Medium erfolgt.

6. Mittel bestehend aus oder enthaltend eine Anthranilsäure gemäß Anspruch 1.

7. Anthranilsäure gemäß Anspruch 1 zur Anwendung als Heilmittel.

**Patentansprüche für den Vertragsstaat: AT**

1. Verfahren zur Herstellung einer basisch substituierten Anthranilsäure der Formel

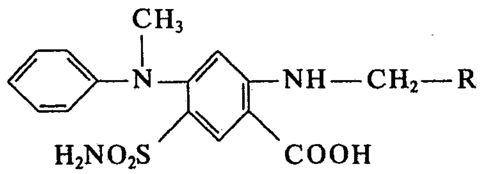

(I)

in der R Furyl, Thienyl oder Phenyl bedeutet und deren physiologisch verträglichen Salzen, dadurch gekennzeichnet, daß man ein Nitril der Formel II

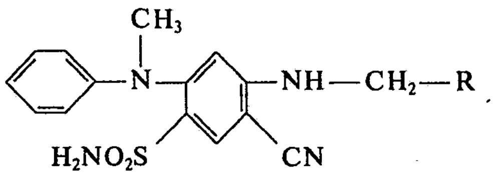

(II)

verseift.

2. Verfahren gemäß Anspruch 1, dadurch gekennzeichnet, daß die Verseifung in einem alkalischen Medium erfolgt.

3. Verfahren gemäß Anspruch 1, dadurch gekennzeichnet, daß R 2-Furyl oder 2-Thienyl ist.

4. Verfahren zur Herstellung eines Salzes einer Anthranilsäure gemäß Anspruch 1 dadurch gekennzeichnet, daß man diese mit einer basischen, kaliumretinierenden Verbindung oder einem basischen Antihypertensivum, umsetzt.

**Claims for the Contracting state: BE, CH, DE, FR, GB, IT, LI, LU, NL, SE**

1. Base-substituted anthranilic acids of the formula I

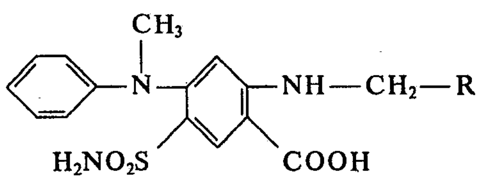

(I)

in which R denotes furyl, thienyl or phenyl, and physiologically acceptable salts thereof.

2. Anthranilic acids as claimed in claim 1, in which R is 2-furyl or 2-thienyl.

3. Salts of an anthranilic acid as claimed in claim 1, formed with a basic, potassium-retaining compound or a basic antihypertensive agent.

4. A process for the preparation of a base-substituted anthranilic acid as claimed in claim 1, which comprises hydrolyzing a nitrile of the formula II

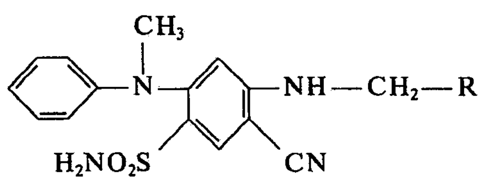

(II)

5. The process as claimed in claim 4, wherein the hydrolysis is carried out in an alkaline medium.

6. An agent composed of or containing an anthranilic acid as claimed in claim 1.

7. Anthranilic acid as claimed in claim 1 for administration as a medicament.

**Claims for the Contracting state: AT**

1. A process for the preparation of a base-substituted anthranilic acid of the formula

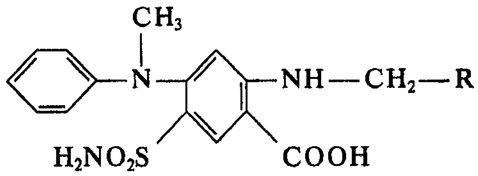

(I)

in which R denotes furyl, thienyl or phenyl, and physiologically acceptable salts thereof, which comprises hydrolyzing a nitrile of the formula II

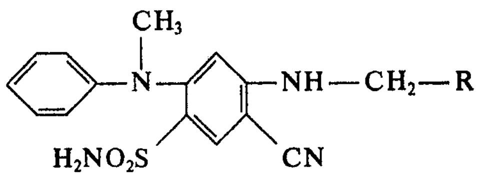

(II)

2. Process as claimed in claim 1, wherein the hydrolysis is carried out in an alkaline medium.
3. Process as claimed in claim 1, in which R is 2-furyl or 2-thienyl.
4. Process for the preparation of a salt of an anthranilic acid as claimed in claim 1, characterized in that the anthranilic acid is reacted with a basic, potassium-retaining compound or a basic antihypertensive agent.

**Revendications pour les Etats contractants: BE, CH, DE, FR, GB, IT, LI, LU, NL, SE**

1. Acides anthraniliques substitués par des groupes basiques, de formule I

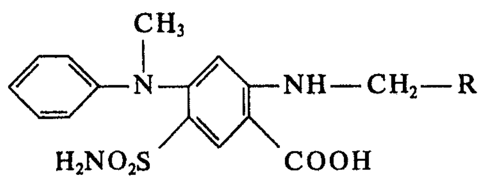

(I)

dans laquelle R représente un groupe furyle, thiényle ou phényle, et leurs sels physiologiquement acceptables.
2. Acides anthraniliques selon la revendication 1, dans lesquels R est le groupe 2-furyle ou 2-thiényle.
3. Sels d'un acide anthranilique selon la revendication 1 avec un composé basique, retenant le potassium ou avec un antihypertenseur basique.
4. Procédé pour la préparation d'un acide anthranilique substitué par un groupe basique selon la revendication 1, caractérisé en ce qu'on saponifie un nitrile de formule II

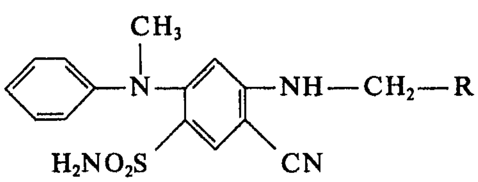

(II)

5. Procédé selon la revendication 4, caractérisé en ce que la saponification a lieu dans un milieu alcalin.
6. Agent constitué de, ou contenant un acide anthranilique selon la revendication 1.
7. Acide anthranilique selon la revendication 1, utilisable comme médicament.

**Revendications pour l'Etat contractant: AT**

1. Procédé pour la préparation d'un acide anthranilique substitué par un groupe basique, de formule

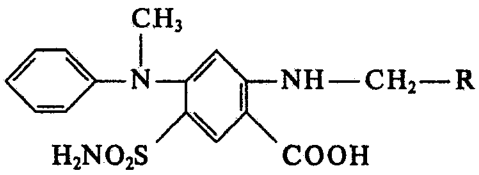

(I)

dans laquelle R représente un groupe furyle, thiényle ou phényle, et ses sels physiologiquement acceptables, caractérisé en ce qu'on saponifie un nitrile de formule II

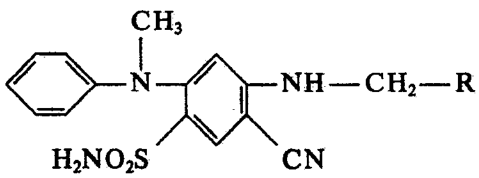

(II)

2. Procédé selon la revendication 1, caractérisé en ce que la saponification a lieu dans un milieu alcalin.

3. Procédé selon la revendication 1, caractérisé en ce que R est le groupe 2-furyle ou 2-thiényle.

4. Procédé pour la préparation d'un acide anthranilique selon la revendication 1, caractérisé en ce qu'on fait réagir cet acide avec un composé basique retenant le potassium ou avec un hypertenseur basique.